(19) Europäisches Patentamt
European Patent Office
Office européen des brevets

(11) Publication number: 0 054 892
B1

(12) # EUROPEAN PATENT SPECIFICATION

(45) Date of publication of patent specification: 18.09.85

(21) Application number: 81110463.7

(22) Date of filing: 15.12.81

(51) Int. Cl.[4]: C 07 D 209/08,
C 07 D 209/10, C 07 D 209/12
// A61K31/40

(54) **Synthesis of indolines.**

(30) Priority: 22.12.80 US 219416

(43) Date of publication of application:
30.06.82 Bulletin 82/26

(45) Publication of the grant of the patent:
18.09.85 Bulletin 85/38

(84) Designated Contracting States:
AT BE CH DE FR GB IT LI LU NL SE

(58) References cited:
US-A-3 565 911

Rodds Chemistry of Carbon Compounds, Vol. IV, part A, p. 446
Indoles, Part One (W.J. Houlihan, ed.), pp. 472-473 (1974)
Bader, A.R. et al., J. American Chemical Soc., vol. 83, pp. 3319-3323 (August 5, 1961)
Schmid, M., et al., Helevetica Chimica Acta, vol. 56, Fasc. 1, Nr. 4, pp. 105-124 (1973)

The file contains technical information submitted after the application was filed and not included in this specification

(73) Proprietor: USV PHARMACEUTICAL CORPORATION
1 Scarsdale Road
Tuckahoe New York (US)

(72) Inventor: Auerbach, Joseph
35 Seacoast Terrace
Brooklyn New York (US)
Inventor: Kantor, Martin Leonard
811 Rockland Avenue
Mamaroneck New York (US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 054 892 B1

**Description**

The present invention relates to a process for the preparation of indolines of the general formula I

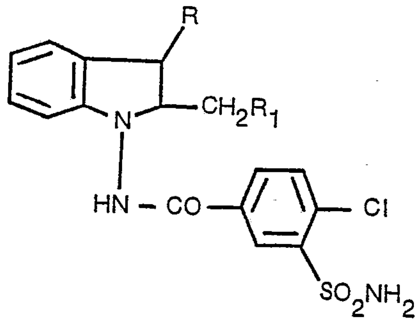

(I)

wherein

R is hydrogen, lower alkyl having 1 to 5 carbon atoms, cycloalkyl having 3 to 7 carbon atoms, phenyl or phenyl substituted with halogen, trifluoromethyl, or lower alkyl or lower alkoxy having 1 to 4 carbon atoms and

$R_1$ is hydrogen, lower alkyl having 1 to 4 carbon atoms, phenyl or phenyl lower alkyl wherein the lower alkyl has 1 to 4 carbon atoms.

The indolines of formula I are pharmaceutically active compounds having antihypertensive activity.

US Patent No. 3 565 911 discloses a process for the preparation of substituted N-amino indoline compounds of the general formula

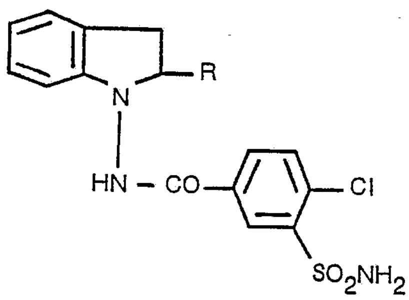

wherein

R represents a substituent selected from the group consisting of:

(a) lower-alkyl containing 1 to 5 carbon atoms inclusive,

(b); phenyl, phenyl substituted by one or more substituents selected from the group consisting of halogen, trifluoromethyl, and lower-alkyl and lower-alkyloxy having one to four carbon atoms inclusive in the lower-alkyl group, and

(c) cyclohexyl.

The preparation of these compounds involves a multi-step process starting with the nitrosation of an indoline appropriately substituted in the 2-position to provide an N-nitroso derivative, followed by the reduction of the nitroso group to give an N-amino indoline, and the reaction of this compound with 3-sulfamyl-4-chlorobenzoyl chloride to give the desired end products.

The yields in these reactions are poor and the synthesis involves the preparation of nitrosamines, compounds which have been reported to be carcinogenic.

It is also known from Indoles, Part One (W. J. Houlihan, ed.) pp. 472—473 (1974); Bader, A. R., et al., J. American Chemical Soc., Vol. 83, pp. 3319—3323 (August 5, 1961) and Schmid, M., et al., Helvetica Chimica Acta, Vol. 56, Fasc. 1, Nr. 4., pp. 105—124 (1973) to cyclize substituted anilines at temperatures of 180 to 200° C in order to prepare indolines, wherein the nitrogen atom is either substituted with a hydrogen atom or a methyl group.

It is the object of the present application to provide an easier process for the preparation of the indolines of formula I. This object is achieved by a process according to claim 1.

Examples of suitable acids include trifluoromethanesulfonic, hydrochloric, sulfuric and phosphoric acids. The cyclization is preferably carried out by heating the compound of formula II and the mineral acid in an inert solvent at a temperature of about 25° C to 100° C. Suitable inert solvents include water, polyhalogenated hydrocarbons, higher boiling ethers and glycol ethers. Preferably, R and $R_1$ are independently hydrogen and lower alkyl.

The compounds of formula II may readily be obtained by treatment of a compound of formula III

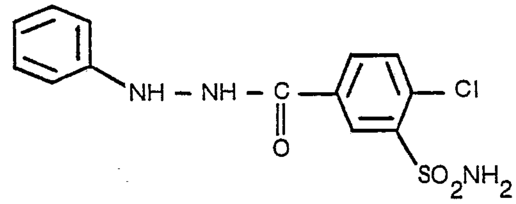

(III)

with an allyl halide of formula IV

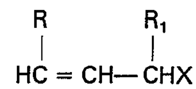

(IV)

wherein

X is halogen, preferably bromine, and

R and $R_1$ are as previously defined and, as noted above, are preferably independently hydrogen and lower alkyl.

The hydrazide of formula III, is readily obtained by treating phenylhydrazine with 3-aminosulfonyl-4-chlorobenzoyl chloride.

The invention will be more fully illustrated in the examples which follow.

## Example I

a) 1-Allyl-1-phenyl-2-(3-sulfamoyl-4-chlorobenzoyl)-hydrazine

A mixture of 3-aminosulfonyl-4-chloro-benzhydrazide (326.6g, 1.0 mole) sodium bicarbonate (420.0g, 2.0 mole), potassium iodide (16.6g, 0.1 mole) and allyl bromide (242.0g, 2.0 mole) in isopropanol (1,500 ml) was stirred at gentle reflux for 20 hours then cooled to 50° and filtered by suction. The cake was washed twice with 100 ml portions of isopropanol. The washes and filtrate were combined and diluted with sufficient water to make a 70% isopropanol solution, then allowed to stir overnight to complete crystallization. The crude product was filtered and washed twice with 100 ml portions of isopropanol-water (70:30). One recrystallization from isopropanol-water (70:30) and clarification with activated charcoal gave pure compound in 80% yield.

b) 1-(4-Chloro-3-sulfamoylbenzamido)-2-methylindoline

A mixture of 1-allyl-1-phenyl-2-sulfamoyl-4-chlorobenzoyl)-hydrazine (36.6g, 0.1 mole) and phosphoric acid, 85% (100 ml) was stirred at 60° for a period of 4 hours. Dilution with water gave crude 1-(4-chloro-3-sulfamoylbenzamido)-2-methylindoline which was purified by recrystallization from isopropanol-n-hexane (60:40).

## Example II

1-(4-Chloro-3-sulfamoylbenzamido)-2-methylindoline

In a 50 ml reaction vessel was placed 1 gram 2.73 mmols of 1-allyl-1-phenyl-2-(3-sulfamoyl-4-chlorobenzoyl)-hydrazine and 10 ml of trifluoromethane sulfonic acid. The reaction mixture was stirred under a nitrogen atmosphere at ambient temperatures. After 15 minutes the solid dissolves and the reaction is allowed to proceed for a total of 4.75 hours. The reaction mixture is worked up by pouring the acid solution into 250 ml of water, extracting the precipitate into ethyl acetate, washing the ethyl acetate several times with water and sodium bicarbonate solution. The ethyl acetate phase is separated and dried with magnesium sulfate and clarified. The isolated product was further purified by dry column chromatography and then crystallized from 50% w/w methanol-acetone.

## Example III

1-(4-Chloro-3-sulfamoylbenzamido)-2-methylindoline

In a 50 ml round bottom was placed 8 grams of Lucas reagent. The reagent is prepared by dissolving 16 grams of anhydrous zinc chloride in 10 ml of concentrated hydrochloric acid. To this reagent is added 100 m g (0.27 mmols) of 1-allyl-1-phenyl-2-(3-sulfamoyl-4-chlorobenzoyl)-hydrazine and stirred under nitrogen for a short time to dissolve the solid. The reatant cyclizes at 60°C. over 6 hours. The reaction is worked up by pouring the cooled reaction mixture into water and extracting the product into ethyl acetate. The ethyl acetate phase is cross washed several times with water and sodium bicarbonate solution. The ethyl acetate is dried with magnesium sulfate, clarified and evaporated to dryness yielding 84 mg of crude 1-(4-chloro-3-sulfamoylbenzamido)-2-methylindoline.

## Example IV

a) 1-Crotyl-1-phenyl-2-(3-sulfamoyl-4-chlorobenzoyl)hydrazine

In a 250 ml round bottomed flask is placed 90 ml of N,N-dimethylformamide and 0.092 mole, 30 grams of 3-amino sulfonyl-4-chloro-benzhydrazide. The mixture was stirred to dissolve the solid, then 18.63

grams, 0.138 mole of crotyl bromide were added. The mixture was heated at 100° for 1.66 hours at which point TLC showed the reaction to be complete. The reaction mixture was cooled and diluted with 200 ml isopropanol. This solution was dripped into a slurry of 1.5 liters of ice and 4 liters of water. The solid which precipitated was collected, washed with water and air dried overnight, yielding the product which could be recrystallized from chloroform/carbon tetrachloride.

The compound gave the correct mass spectrum NMR and CMR analysis. Carbon magnetic resonance shows cis/trans relative ratio of the crotyl side chain to be approximately 10:90.

b) 1-(4-Chloro-3-sulfamoylbenzamido)-2,3-dimethyl indoline

1.97 mmol, .0175 g 1-crotyl-1-phenyl-2-[(3-sulfamoyl-4-chloro-benzoyl)]-hydrazine was mixed with 15 ml of phosphoric acid and kept at 100°C. for 45 minutes at which point thin layer chromatography showed that starting material was consumed. The mixture was cooled and poured into water, 40 ml, and extracted with ethyl acetate, 40 ml, 2 times. The ethyl acetate was cross washed with water, then 1 mole sodium bicarbonate, the brine. The ethyl acetate was dried and the solution taken to dryness. The residue was filtered through silica gel, eluting with ethyl acetate-hexane and the eluent was taken to dryness giving the product. The structure is supported by mass spectrum and NMR analysis.

**Claims**

1. A process for the preparation of indolines of the general formula I

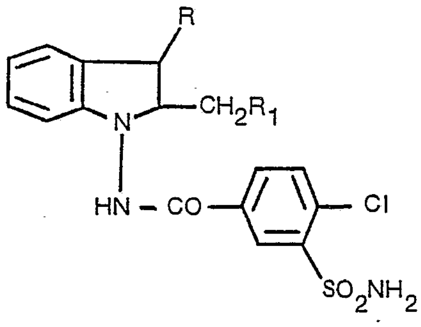

(I)

wherein

R is hydrogen, lower alkyl having 1 to 5 carbon atoms, cycloalkyl having 3 to 7 carbon atoms, phenyl or phenyl substituted with halogen, trifluoromethyl, or lower alkyl or lower alkoxy having 1 to 4 carbon atoms and

$R_1$ is hydrogen, lower alkyl having 1 to 4 carbon atoms, phenyl or phenyl lower alkyl wherein the lower alkyl has 1 to 4 carbon atoms, which comprises cyclizing a compound of the formula II

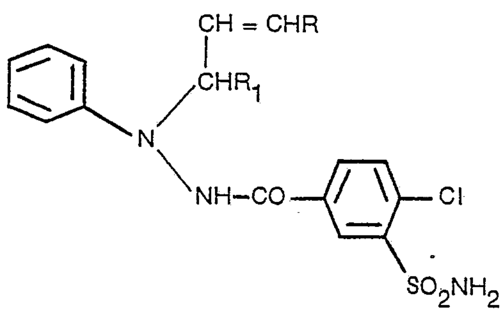

(II)

wherein

R and $R_1$ are as defined above, in the presence of a Lewis or a Bronsted acid or mixture thereof.

2. The process according to Claim 1 wherein R and $R_1$ are independently hydrogen or lower alkyl.

3. The process according to Claim 1 or 2 wherein R is hydrogen.

4. The process according to any of Claims 1—3 wherein $R_1$ is hydrogen or an alkyl group having 1 to 4 carbon atoms.

5. The process according to Claim 4 wherein $R_1$ is hydrogen.

6. The process according to any of Claims 1—5 wherein the acid is phosphoric acid.

7. The process according to any of Claims 1—5 wherein the acid is hydrochloric acid.

8. The process according to any of Claims 1—7 wherein the product is 1-(4-chloro-3-sulfamoylbenzamido)-2-methylindoline.

**Patentansprüche**

1. Verfahren zur Herstellung von Indolinen der allgemeinen Formel I

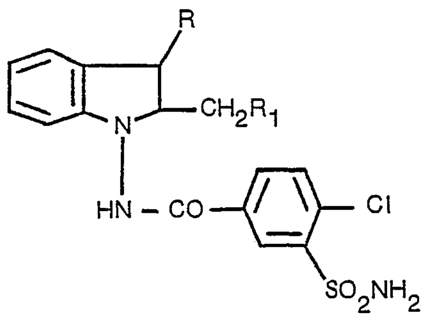

(I)

worin

R Wasserstoff, Niedrigalkyl mit 1 bis 5 Kohlenstoffatomen, Cycloalkyl mit 3 bis 7 Kohlenstoffatomen, Phenyl oder Phenyl, substituiert mit Halogen, Trifluormethyl oder Niedrigalkyl oder Niedrigalkoxy mit 1 bis 4 Kohlenstoffatomen, und

$R_1$ Wasserstoff, Niedrigalkyl mit 1 bis 4 Kohlenstoffatomen, Phenyl oder Phenyl-Niedrigalkyl, worin das Niedrigalkyl 1 bis 4 Kohlenstoffatome hat, ist,

welches das Zyklisieren einer Verbindung der Formel II

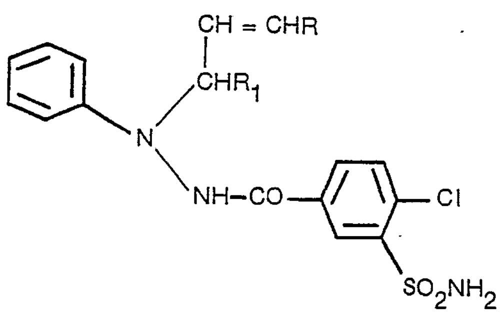

(II)

worin

R und $R_1$ die vorstehende Bedeutung besitzen, in Gegenwart einter Lewis- oder Bronsted-Säure oder Mischungen davon umfaßt.

2. Verfahren nach Anspruch 1, worin R und $R_1$ unabhängig voneinander Wasserstoff oder Niedrigalkyl sind.

3. Verfahren nach Anspruch 1 oder 2, worin R Wasserstoff ist.

4. Verfahren nach einem der Ansprüche 1 bis 3, worin $R_1$ Wasserstoff oder eine Alkylgruppe mit 1 bis 4 Kohlenstoffatomen ist.

5. Verfahren nach Anspruch 4, worin $R_1$ Wasserstoff ist.

6. Verfahren nach einem der Ansprüche 1 bis 5, worin die Säure Phosphorsäure ist.

7. Verfahren nach einem der Ansprüche 1 bis 5, worin die Säure Salzsäure ist.

8. Verfahren nach einem der Ansprüche 1 bis 7, worin das Produkt 1-(4-Chlor-3-sulfamoylbenzamido)-2-methylindolin ist.

**Revendications**

1. Procédé pour la préparation d'indolines de la formule générale I

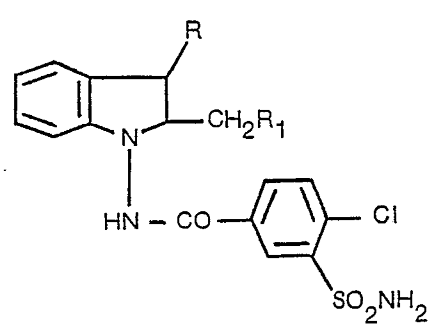

(I)

dans laquelle:

R est l'hydrogène, une groupe alkyle inférieur ayant 1 à 5 atomes de carbone, cycloalkyle ayant 3 à 7

atomes de carbone, phényle ou phényle substitué par un halogène, un groupe trifluorométhyle ou un groupe alkyle inférieur ou alcoxyle inférieur ayant 1 à 4 atomes de carbone, et

$R_1$ est l'hdrogène, un groupe alkyle inférieur ayant 1 à 4 atomes de carbone, phényle ou phényl-alkyle inférieur dans lequel le groupe alkyle inférieur a 1 à 4 atomes de carbone, qui consiste à cycliser un composé de la formule II

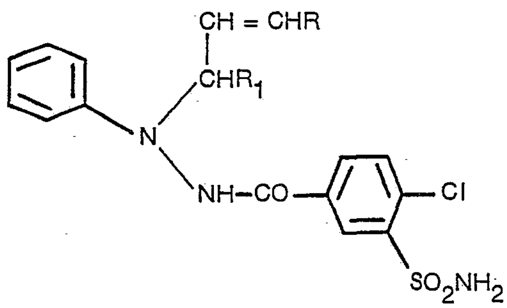

(II)

dans lacquelle:

R et $R_1$ sont comme définis ci-dessus, en présence d'un acide de Lewis ou de Bronsted ou de mélanges de ceux-ci.

2. Procédé selon la revendicatioon 1, dans lequel R et $R_1$ sont indépendamment l'hydrogène ou un groupe alkyle inférieur.

3. Procédé selon l'une des revendications 1 et 2, dans lequel R est l'hydrogène.

4. Procédé selon l'une quelconque des revendications 1 à 3, dans lequel $R_1$ est l'hydrogène ou un groupe alkyle ayant 1 à 4 atomes de carbone.

5. Procédé selon la revendication 4, dans lequel $R_1$ est l'hydrogène.

6. Procédé selon l'une quelconque des revendications 1 à 5, dans lequel l'acide est l'acide phosphorique.

7. Procédé selon l'une quelconque des revendications 1 à 5, dans lequel l'acide est l'acide chlorhydrique.

8. Procédé selon l'une quelconque des revendications 1 à 7, dans lequel le produit est la 1-(4-chloro-3-sulfamoylbenzamido)-2-méthylindoline.